(19) Europäisches Patentamt
European Patent Office
Office européen des brevets

(11) Veröffentlichungsnummer: **0 005 749**
**B1**

(12) **EUROPÄISCHE PATENTSCHRIFT**

(45) Veröffentlichungstag der Patentschrift: **30.03.83**

(51) Int. Cl.³: **C 07 C175/00**, C 07 D317/26

(21) Anmeldenummer: **79101401.2**

(22) Anmeldetag: **08.05.79**

# (54) Cyclohexenderivate, Verfahren zu deren Herstellung, sowie deren Verwendung.

(30) Priorität: **02.06.78 CH 6073/78**
**29.03.79 CH 2921/79**

(43) Veröffentlichungstag der Anmeldung: **12.12.79 Patentblatt 79/25**

(45) Bekanntmachung des Hinweises auf die Patenterteilung: **30.03.83 Patentblatt 83/13**

(84) Benannte Vertragsstaaten: **AT BE CH DE FR GB IT NL**

(56) Entgegenhaltungen:
**FR A 2 357 541**
**US A 4 098 827**

(73) Patentinhaber: **F. HOFFMANN-LA ROCHE & CO. Aktiengesellschaft**
**CH-4002 Basel (CH)**

(72) Erfinder: **Mayer, Hans Johann, Dr.**
**Obere Hofackerstrasse 5**
**CH-4414 Füllinsdorf (CH)**
Erfinder: **Müller, Robert Karl, Dr.**
**Wettsteinallee 145**
**CH-4058 Basel (CH)**

(74) Vertreter: **Aschert, Hubert et al**
**Postfach 601 Grenzacherstrasse 124**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

# Cyclohexenderivate, Verfahren zu deren Herstellung, sowie deren Verwendung

Die Erfindung betrifft Cyclohexenderivate der allgemeinen Formel

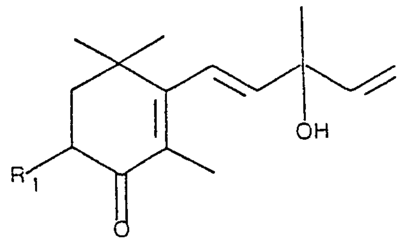

(I)

in der $R_1$ eine in die Hydroxygruppe überführbare Acyloxygruppe oder Aethergruppe darstellt, ein Verfahren zur Herstellung dieser Verbindungen, sowie die Verwendung dieser Verbindungen zur Herstellung von Astaxanthin.

Die Formel I wie auch die nachfolgenden Formeln II, III und IV umfassen sowohl racemische als auch optisch aktive Verbindungen.

Racemische Verbindungen der Formel I können durch die allgemeine Formel

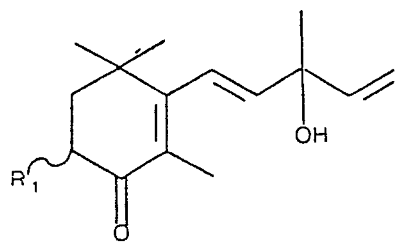

(IAB)

optisch aktive (S)-Verbindungen der Formel I durch die allgemeine Formel

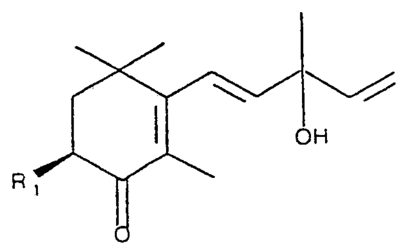

(IA)

und optisch aktive (R)-Verbindungen der Formel I durch die allgemeine Formel

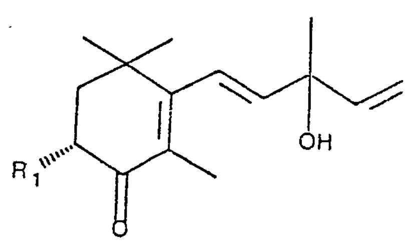

(IB)

gekennzeichnet werden.

Das Zeichen ∼ bedeutet, dass die Gruppe $R_1$ sowohl vor als auch hinter der Ebene des Moleküls liegen kann. Der Keil ▶ bedeutet, dass die mit diesem Zeichen verbundene Gruppe $R_1$ vor der Ebene des Moleküls, die unterbrochene ⁞⁞⁞⁞ Linie, dass die dieser angeschlossene Gruppe $R_1$ hinter der Ebene des Moleküls liegt.

Die Acyloxygruppe $R_1$ kann sich beispielsweise von niederen wie höheren Alkan- und Alkencarbonsäuren ableiten. Die niederen Glieder enthalten bis zu 6, die höheren bis zu 20 Kohlenstoffatome. Beide Glieder sind vorzugsweise durch Halogen, Alkoxy oder Aryloxy substituiert, denn die durch Halogen, Alkoxy oder Aryloxy substituierten Acyloxygruppen können im vorliegenden System besonders

einfach zur Hydroxygruppe verseift werden, ohne dass diese bei der Verseifung teilweise in die Oxogruppe umgewandelt wird. Die Acyloxygruppe kann sich auch von einer optisch aktiven, als Spaltungsreagens verwendbaren Säure, z. B. der (−)-Camphersäure ableiten.

Als Beispiele für die in Hydroxy überführbaren Acyloxygruppen $R_1$ können genannt werden :

— von den niederen Gliedern die Acetoxy-, Propionyloxy-, Butyryloxy-, Valeryloxy-, Pivaloyloxy- und Capryloxygruppe

sowie die Monochloracetoxy-, Dichloracetoxy-, Aethoxyacetoxy- und Phenoxyacetoxygruppe

— von den höheren Gliedern die Palmitoyloxy-, Stearoyloxy- und Oleoyloxy-gruppe.

Von diesen ist die α-Chlor-, α-Aethoxy- und α-Phenoxyacetoxy-gruppe bevorzugt.

$R_1$ umfasst derner die (−)-Camphanoyloxygruppe.

Als in die Hydroxygruppe überführbare Aethergruppen kommen solche in Frage, welche durch Hydrolyse leicht in die Hydroxygruppe übergeführt werden, beispielsweise die 1-Methoxy-1-methyläthoxygruppe oder die tert. Butoxygruppe.

Das Verfahren zur Herstellung der Polyenverbindungen der Formel I ist dadurch gekennzeichnet, dass man eine rac. oder opt. aktive Polyenverbindung der allgemeinen Formel

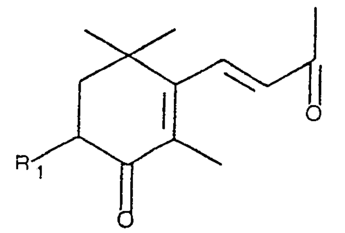

(II)

in der $R_1$ die oben gegebene Bedeutung hat, vinyliert, oder äthinyliert und partiell hydriert.

Es war zu erwarten, dass bei den vorstehenden Reaktionen ein Gemisch von Reaktionsprodukten bestehend aus an der Seitenkette und/oder am Ring vinylierten bzw. äthinylierten Verbindungen anfallen würde. Es war ferner zu befürchten, dass die Gruppe $R_1$ mit dem Vinylierungs- bzw. Aethinylierungsreagenz reagieren würde. Beides ist jedoch überraschenderweise nicht der Fall.

Lässt man nämlich auf die in einem aprotischen Lösungsmittel gelöste Ausgangsverbindung der Formel II in einem unter 0° liegenden Temperaturbereich ein Vinylmagnesiumhalogenid oder ein Aethinylmagnesiumhalogenid einwirken, so erhält man praktisch ausschliesslich die gewünschte seitenkettenverlängerte Verbindung der Formel I.

Von den aprotischen Lösungsmitteln sind insbesondere geeignet :

— Tetrahydrofuran
— Diäthylenglykol-dimethyläther und
— Aethylenglykol-dimethyläther.

Der für die Vinylierung günstige Temperaturbereich liegt zwischen etwa − 30 und etwa − 100 °C.

Der für die Aethinylierung günstige Temperaturbereich liegt zwischen etwa − 30 und + 30 °C.

Von den Vinylierungs- und Aethinylierungsmitteln kommen vornehmlich das Vinylmagnesiumchlorid und -bromid, sowie das Aethinylmagnesium-chlorid und -bromid in Frage.

Die bei der Aethinylierung einer Verbindung der Formel II primär erhältlichen Verbindungen der allgemeinen Formel

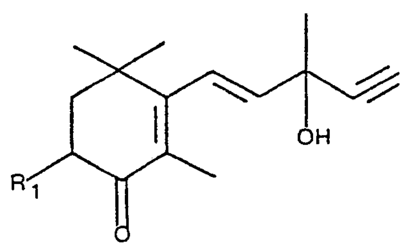

(III)

werden durch Partialhydrierung in die gewünschten Endprodukte der Formel I umgewandelt.

Die Partialhydrierung der Aethinyl- zur Vinyl-gruppe kann in an sich bekannter Weise mit Hilfe eines partiell vergifteten Palladiumkatalysators [Lindlarkatalysator] in einem Lösungsmittel, wie Benzol, Toluol, Essigsäureäthylester oder auch in einem Alkanol, wie Methanol oder Isopropanol durchgeführt werden und zwar zweckmässig bei Normaldruck und Raumtemperatur.

Die neuen Polyenverbindungen der allgemeinen Formel I sind Schlüsselverbindungen für die Herstellung von racemischem und optisch aktivem Astaxanthin. Das 3(S), 3'(S)-Astaxanthin und das 3(R), 3'(R)-Astaxanthin sind Naturprodukte. Sie können, wie weiter unten beschrieben, aus den Verbindungen der Formeln IA bzw. IB synthetisiert werden. Des weiteren ist auch die Mesoform des optisch aktiven Astaxanthin, das 3(R), 3'(S)-Astaxanthin aus den Schlüsselverbindungen IA und IB erhältlich. Astaxanthin ist ein zum Färben von Lebensmitteln begehrter Rotfarbstoff, der in der Natur zwar weit verbreitet ist, aber, nicht zuletzt wegen der geringen Konzentration, nur mit erheblichem Aufwand und unbefriedigender Ausbeute isoliert werden kann.

Die als Ausgangssubstanzen eingesetzten Verbindungen der Formel II können z. B. wie folgt hergestellt werden :

Die rac. Ausgangssubstanzen der Formel IIAB sind ausgehend von 4-Oxo-β-ionon (VI) über folgende Reaktionsstufen

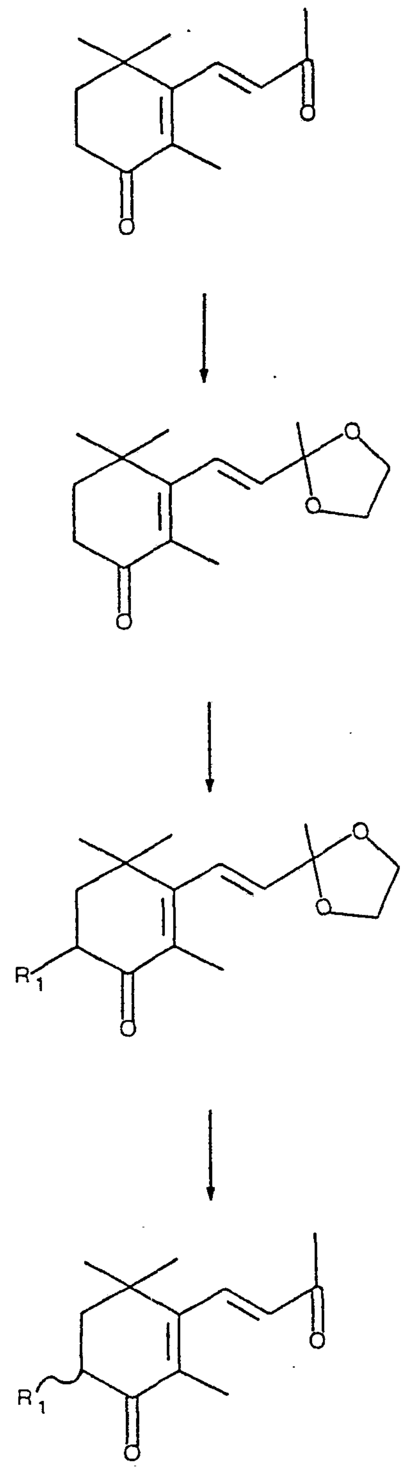

(VI)

(VII)

(VIII)

(IIAB)

erhältlich ($R_1$ = Acyloxy).

Die optisch aktiven Ausgangssubstanzen der Formel IIA und IIB, in welchen $R_1$ eine in die Hydroxygruppe überführbare Acyloxygruppe darstellt, sind aus den rac. Ausgangsverbindungen der Formel IIAB über folgende Zwischenstufen

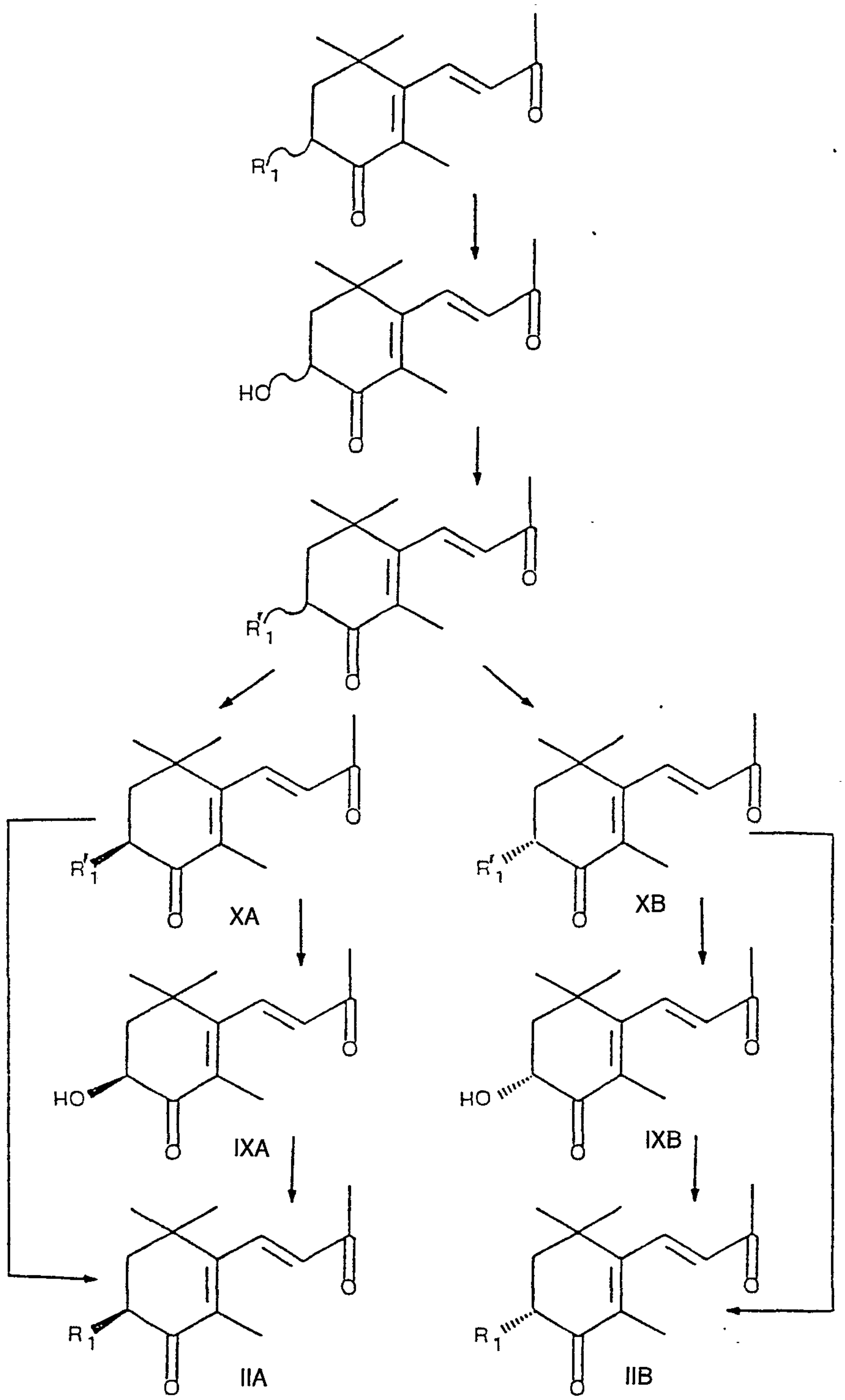

IIAB

IXAB

XAB

XA

XB

IXA

IXB

IIA

IIB

zugänglich.

$R'_1$ = Acyloxyrest einer zur Racematspaltung geeigneten Säure, z. B. der (−)-Camphansäure.

Die vorstehend skizzierten Reaktionsschritte können wie folgt durchgeführt werden.

Herstellung von rac. 3-Acyloxy-4-oxo-β-ionon (IIAB) aus 4-Oxo-β-ionon (VI) :

4-Oxo-β-ionon (VI) wird in Gegenwart von p-Toluolsulfonsäure mit 1,2 Moläquivalenten Orthoameisensäureäthylester zu 4-Oxo-β-ionon-9-äthylenketal (VII) umgesetzt. Das erhaltene Ketal (VII) wird durch Behandeln mit einem Acylierungsmittel, z. B. mit Bleitetraacetat, in einem Lösungsmittel, wie Toluol, in der Siedehitze in rac. 3-Acyloxy-4-oxo-β-ionon-9-äthylenketal (VIII) umgewandelt und anschliessend durch Schütteln mit Wasser-Methanol-Eisessig im Verhältnis 10 : 10 : 1 bei Raumtemperatur zu dem gewünschten rac. 3-Acyloxy-4-oxo-β-ionon (IIAB) deketalisiert.

Herstellung von 3-(S)-Acyloxy-4-oxo-β-ionon (IIA) aus rac. 3-Acyloxy-4-oxo-β-ionon (IIAB) :

rac. 3-Acyloxy-4-oxo-β-ionon (IIAB) wird in einem Alkanol unter Sauerstoffausschluss mit Hilfe von wässerigem Alkali, insbesondere mit 1 n Natronlauge, zu rac. 3-Hydroxy-4-oxo-β-ionon (IXAB) verseift. Der erhaltene Alkohol (IXAB) wird anschliessend in seine Enantiomeren aufgetrennt. Um diese Auftrennung durchführen zu können, verestert man den Alkohol mit einer geeigneten Säure, vorzugsweise mit (−)-Camphansäure. Die Veresterung mit (−)-Camphansäurechlorid in Pyridin liefert ein kristallines Estergemisch (XAB), aus dem beispielsweise durch fraktionierte Kristallisation aus Essigsäureäthylester/Hexan das gewünschte 3(S)-(−)-Camphanoyloxy-4-oxo-β-ionon (XA) sowie das 3(R)-(−)-Camphanoyloxy-4-oxo-β-ionon (XB) in hoher Reinheit isoliert werden können. Die Diastereomeren (XA) und (XB) sind Vertreter der Ausgangsverbindungen der Formeln IIA und IIB, in denen $R_1$ die (−)-Camphanoyloxygruppe darstellt. Die Camphansäurederivate XA und XB können demnach unmittelbar als Ausgangssubstanzen für die erfindungsgemässe Vinylierung oder Aethinylierung eingesetzt werden.

Die erhaltenen Verbindungen der Formeln XA und XB können aber, um das Spaltungsreagenz, die (−)-Camphansäure, sofort zurückzugewinnen, auch zunächst zu 3(S)-Hydroxy-4-oxo-β-ionon (IXA) bzw. zu 3(R)-Hydroxy-4-oxo-β-ionon (IXB) verseift und anschliessend durch Einführung einer der eingangs erwähnten Acylgruppen erneut zu 3(S)-Acyloxy-4-oxo-β-ionon (IIA) bzw. zu 3(R)-Acyloxy-4-oxo-β-ionon (IIB) verestert werden.

Diejenigen Ausgangsmaterialien der Formel II, in welchen $R_1$ eine in die Hydroxygruppe überführbare Aethergruppe darstellt, sind in einfacher Weise durch Verätherung von 3-Hydroxy-4-oxo-β-ionon erhältlich.

Wie eingangs erwähnt, stellen die neuen Polyenverbindungen der Formel I Schlüsselverbindungen für die Herstellung von rac. und optisch aktivem Astaxanthin dar.

Für die Synthese von Astaxanthin werden die Verbindungen der Formel I entweder durch Isomerisieren und Oxydieren, oder durch Halogenierung und Umsetzen mit einem Triarylphosphin in einen rac. oder optisch aktiven $C_{15}$-Baustein der allgemeinen Formel

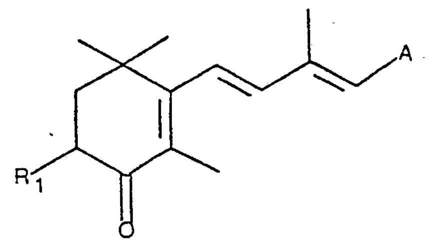

(IV)

in der $R_1$ die oben gegebene Bedeutung hat, und A entweder die Formylgruppe oder eine Triarylphosphoniummethylgruppe der Formel $—CH_2—P[X]_3^{\oplus}Y^{\ominus}$ bedeutet, worin X einen Arylrest und Y das Anion einer anorganischen oder organischen Säure bezeichnet, umgewandelt und anschliessend mit einem $C_{10}$-Baustein der allgemeinen Formel

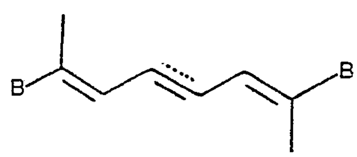

(V)

in der B entweder eine Triarylphosphoniummethylgruppe der Formel $—CH_2—P[X]_3^{\oplus}Y^{\ominus}$ oder die Formylgruppe bezeichnet, und die punktierte Linie gegebenenfalls eine zusätzliche Kohlenstoff-Kohlenstoff-Bindung bezeichnet,

zu rac. oder optisch aktivem, in Stellung 3 und 3′ mit einer Acyl- oder Aethergruppe geschütztem Astaxanthin oder 15,15′-Didehydroastaxanthin oder unmittelbar zu Astaxanthin bzw. 15,15′-Didehydroastaxanthin kondensiert.

Erhaltene Acylate können durch Verseifung und, falls erforderlich, durch Partialhydrierung in rac. und optisch aktives Astaxanthin übergeführt werden.

Die Verbindungen der allgemeinen Formel IV und V werden nach der von Wittig angegebenen Arbeitsweise in Gegenwart eines säurebindenden Mittels, z. B. in Gegenwart eines Alkalimetallalkoholates wie Natriummethylat, Lithiumcarbonat oder Natriumbicarbonat, oder in Gegenwart eines gegebenenfalls alkylsubstituierten Alkylenoxyds, insbesondere in Gegenwart von Aethylenoxyd oder 1,2-Butylenoxyd, gegebenenfalls in einem Lösungsmittel, z. B. in einem Alkanol wie Isopropanol, oder in einem halogenierten Kohlenwasserstoff wie Methylenchlorid, oder auch in Dimethylformamid miteinander umgesetzt.

Wird die bei der Verknüpfung der Kondensationskomponenten IV und V angewandte, vorstehend

näher beschriebene Wittig-Reaktion in Gegenwart eines protischen Lösungsmittels wie in Beispiel 5 exemplifiziert, durchgeführt, so wird unter den Kondensationsbedingungen eine vorhandene hydrolytisch leicht entfernbare Acylgruppe abgespalten und man erhält unmittelbar rac. oder optisch aktives Astaxanthin bzw. 15,15'-Didehydroastaxanthin.

Führt man dagegen die Kondensation nach Wittig in einem aprotischen Lösungsmittel, z. B. in Diäthyläther, aus wie in Beispiel 6, so bleiben auch leicht hydrolysierbare Acylgruppen erhalten.

Von den Acyloxygruppen $R_1$ in Verbindungen der Formel IV nehmen die durch Chlor oder Phenoxy substituierten Acyloxygruppen eine Vorzugsstellung ein. Die Trichlor- [oder Trifluor-] acetoxy-gruppe z. B. hat sich, weil zu labil, als weniger geeignet erwiesen.

Die aus dem Astaxanthinderivat durch Hydrolyse entfernbaren Acylreste der Acyloxygruppen können in an sich bekannter Weise abgespalten werden. Wegen der Empfindlichkeit des Moleküls führt man die Hydrolyse der Acyloxygruppen unter möglichst schonenden Bedingungen durch. Die oben genannten Reste lassen sich ohne weiteres durch Behandeln mit schwachen Alkalien in einem zwischen etwa − 30 und etwa + 50 °C liegenden Temperaturbereich zur Hydroxygruppe verseifen. Die mono- und di-Chloracetylgruppen lassen sich bereits durch blosses Erhitzen in Wasser oder in einem wässrigen Alkanol hydrolysieren.

Die Aethergruppen können durch Hydrolyse in die Hydroxygruppen übergeführt werden. Beispielsweise kann die 1-Methoxy-1-methyl-äthoxygruppe durch Behandeln mit wässriger Salzsäure, oder die tert. Butoxygruppe durch Behandlung mit zinkchlorid in die Hydroxygruppe übergeführt werden.

## Beispiel 1

### rac. 5-[4-Acetoxy-3-oxo-2,6,6-trimethyl-cyclohex-1-en-1-yl]-3-methyl-penta-1,4-dien-3-ol

2,2 g rac. 3-Acetoxy-4-oxo-β-ionon werden in 40 ml abs. Tetrahydrofuran gelöst. Die Lösung wird unter Rühren bei etwa − 75 °C unter Argon im Verlauf von 30 Minuten tropfenweise mit 1,1 Moläquivalenten einer ca. 1 molaren Lösung von Vinylmagnesiumchlorid in Tetrahydrofuran versetzt. Die kalte Reaktionslösung wird nach Zugabe von 5 ml einer konz. wässerigen Ammoniumchloridlösung mit Aether extrahiert. Der Aetherextrakt wird getrocknet und unter vermindertem Druck eingedampft. Das zurückbleibende rac. 5-[4-Acetoxy-3-oxo-2,6,6-trimethyl-cyclohex-1-en-1-yl]-3-methyl-penta-1,4-dien-3-ol wird durch Adsorption an Kieselgel [Elutionsmittel : Aether/Hexan 4 : 1] gereinigt. Farbloses Oel. $n_D^{24}$ = 1,511 8.

Das als Ausgangsverbindung eingesetzte rac. 3-Acetoxy-4-oxo-β-ionon kann z. B. wie folgt hergestellt werden :

a) 4-Oxo-β-ionon-9-äthylen-ketal

79,45 g 4-Oxo-β-ionon werden in 68,50 g Orthoameisensäureäthylester eingetragen und nach Zugabe von 35,85 g Aethylenglykol unter Rühren mit 385 mg p-Toluolsulfonsäure versetzt. Das Gemisch wird zunächst 30 Minuten und nach Zugabe von 1,93 g Natriumcarbonat weitere 5 Minuten gerührt, danach in 250 ml einer konz. wässerigen Natriumchloridlösung, 250 ml Wasser und 500 g Eis eingetragen und mit insgesamt 2 000 ml Aether extrahiert. Der Aetherextrakt wird zunächst mit einer halbkonzentrierten, danach mit einer konzentrierten wässerigen Natriumchloridlösung gewaschen, über Natriumsulfat getrocknet und unter vermindertem Druck eingedampft. Das zurückbleibende 4-Oxo-β-ionon-9-äthylen-ketal schmilzt nach dem Umkristallisieren aus Pentan bei 35-37 °C.

b) rac. 3-Acetoxy-4-oxo-β-ionon-9-äthylen-ketal rac. 3-Acetoxy-4-oxo-β-ionon

37,5 g 4-Oxo-β-ionon-9-äthylen-ketal und 133,0 g Bleitetraacetat werden nach Zugabe von 600 ml Toluol 8 Stunden unter Rückflussbedingungen zum Sieden erhitzt. Die Reaktionslösung wird nach Abkühlen filtriert. Das nach Abdampfen des Filtrats zurückbleibende 3-Acetoxy-4-oxo-β-ionon-9-äthylenketal wird zur Entketalisierung in 525 ml Wasser-Methanol-Eisessig 10 : 10 : 1 aufgenommen und 12 Stunden gerührt, danach in 1 000 ml Eis/Wasser eingetragen und mit insgesamt 1 200 ml Aether extrahiert. Der Aetherextrakt wird mit einer gesättigten, wässerigen Natriumchloridklösung, mit einer gesättigten, wässerigen Natriumhydrogencarbonatlösung und danach nochmals mit einer gesättigten, wässerigen Natriumchloridlösung gewaschen, über Natriumsulfat getrocknet und unter vermindertem Druck eingedampft. Das zurückbleibende rac. 3-Acetoxy-4-oxo-β-ionon schmilzt nach Umkristallisation aus Aether/Hexan bei 65-67 °C.

## Beispiel 2

### 5-[4(S)-Acetoxy-3-oxo-2,6,6-trimethyl-cyclohex-1-en-1-yl]-3-methyl-penta-1,4-dien-3-ol

1,5 g 3(S)-Acetoxy-4-oxo-β-ionon werden in 25 ml abs. Tetrahydrofuran gelöst. Die Lösung wird unter Rühren bei etwa − 78 °C unter Argon im Verlauf von 15 Minuten tropfenweise mit 6,3 ml einer 0,95 molaren Lösung von Vinylmagnesiumchlorid in Tetrahydrofuran versetzt. Die kalte Reaktionslösung wird 30 Minuten nachgerührt, danach in 50 g Eis/Wasser eingetragen und nach Zugabe von 50 ml einer

konzentrierten, wässerigen Ammoniumchloridlösung mit insgesamt 300 ml Aether extrahiert. Der Aetherextrakt wird zweimal mit je 100 ml einer halbgesättigten, wässerigen Natriumchloridlösung gewaschen, über Natriumsulfat getrocknet und unter vermindertem Druck eingedampft. Das zurückbleibende 5-[4(S)-Acetoxy-3-oxo-2,6,6-trimethyl-cyclohex-1-en-1-yl]-3-methyl-penta-1,4-dien-3-ol wird durch Adsorption an Kieselgel [Elutionsmittel Aether/Hexan 4 : 1] gereinigt. Farbloses, viskoses Oel. $n_D^{24}$ = 1,497 8.

Das als Ausgangsverbindung eingesetzte optisch aktive 3(S)-Acetoxy-4-oxo-β-ionon kann z. B. wie folgt dargestellt werden :

a) rac. 3-Hydroxy-4-oxo-β-ionon

21,1 g rac. 3-Acetoxy-4-oxo-β-ionon werden in 80 ml Methanol gelöst. Die Lösung wird unter Argon bei − 5 °C im Verlauf von 30 Minuten tropfenweise mit 80 ml 1 n Natronlauge versetzt. Das Reaktionsgemisch wird in Eis/Wasser eingetragen und mit Aether extrahiert. Der Aetherextrakt wird gewaschen, getrocknet und anschliessend unter vermindertem Druck eingedampft. Das zurückbleibende rac. 3-Hydroxy-4-oxo-β-ionon schmilzt nach dem Umkristallisieren aus Aether/Hexan bei 49,5-51 °C.

b) 3(−)-Camphanoyloxy-4-oxo-β-ionon [Diastereomerengemisch]

8,88 g rac. 3-Hydroxy-4-oxo-β-ionon werden in 40 ml Pyridin gelöst. Die Lösung wird unter Feuchtigkeitsausschluss bei 0° mit 10,4 g (−)-Camphansäurechlorid versetzt. Das Reaktionsgemisch wird zunächst 30 Minuten bei 0°, danach 3 Stunden bei + 15 °C gerührt, anschliessend in Eis/Wasser eingetragen und mit Chloroform extrahiert. Noch vorhandene Reste Pyridin werden durch Zugabe von verd. Salzsäure bei 0° gebunden. Das nach Abdampfen des Lösungsmittels zurückbleibende Gemisch der beiden diastereomeren (−)-Camphansäureester, ein zähflüssiges Oel, wird wie nachstehend beschrieben, in die Diastereomeren aufgetrennt.

c) 3(S) und 3(R)-(−)-Camphanoyloxy-4-oxo-β-ionon

Das vorstehend erhaltene Diastereomerengemisch wird durch Chromatographie an Kieselgel [Elutionsmittel : Aether/Hexan 4 : 1], oder durch fraktionierte Kristallisation aus Essigsäureäthylester/Hexan in 3(S)-(−)-Camphanoyloxy-4-oxo-β-ionon [Fp. 119-120 °C] und 3(R)-(−)-Camphanoyloxy-4-oxo-β-ionon [Fp. 124-126 °C] aufgetrennt.

d) 3(S)-Hydroxy-4-oxo-β-ionon

2,01 g 3(S)-(−)-Camphanoyloxy-4-oxo-β-ionon werden nach Zugabe von 30 ml abs. Methanol unter Sauerstoffausschluss und Rühren bei 0° im Verlauf von 15 Minuten tropfenweise mit 5,2 ml 1 n Natronlauge versetzt. Das Reaktionsgemisch wird anschliessend 3 Stunden bei Raumtemperatur gerührt, danach in 100 g Eis/Wasser eingetragen und nach Zugabe von 100 ml konzentrierter, wässeriger Natriumchloridlösung mit 300 ml Aether extrahiert. Der Aetherextrakt wird zweimal mit je 100 ml einer halbkonzentrierten, wässerigen Natriumchloridlösung gewaschen, über Natriumsulfat getrocknet und unter vermindertem Druck eingedampft. Das zurückbleibende 3(S)-Hydroxy-4-oxo-β-ionon schmilzt nach Reinigung durch Adsorption an Kieselgel [Elutionsmittel Aether/Hexan 4 : 1] und Kristallisation aus Aether/Hexan bei 36-37 °C, $[\alpha]_D$ = − 97,2° [in Aethanol].

Die analoge Verseifung von 3(R)-(−)-Camphanoyloxy-4-oxo-β-ionon liefert 3(R)-Hydroxy-4-oxo-β-ionon, Fp : 35-36 °C, $[\alpha]_D$ = + 96,2° [in Aethanol].

e) 3(S)-Acetoxy-4-oxo-β-ionon

160 mg 3(S)-Hydroxy-4-oxo-β-ionon werden in 1,6 ml Pyridin gelöst. Die Lösung wird nach Zugabe von 0,4 ml Essigsäureanhydrid 16 Stunden bei Raumtemperatur gerührt. Das Reaktionsgemisch wird danach in 20 g Eis/Wasser eingetragen und dreimal mit je 30 ml Aether extrahiert. Die vereinigten Aetherextrakte werden unter Zugabe von Eis zweimal mit je 20 ml einer halbkonzentrierten, wässerigen Natriumchloridlösung, zweimal mit je 20 ml 1 n Salzsäure und einmal mit 20 ml einer halbkonzentrierten, wässerigen Natriumchloridlösung gewaschen, über Natriumsulfat getrocknet und unter vermindertem Druck eingedampft. Das zurückbleibende 3(S)-Acetoxy-4-oxo-β-ionon schmilzt nach dem Umkristallisieren aus Aether/Hexan bei 64-66 °C. $[\alpha]_D$ = − 97,3° (in Aethanol).

## Beispiel 3

5-[4(R)-(−)-Camphanoyloxy-3-oxo-2,6,6-trimethyl-cyclohex-1-en-1-yl]-3-methyl-penta-1,4-dien-3-ol

4,02 g 3(R)-(−)-Camphanoyloxy-4-oxo-β-ionon werden in 70 ml Tetrahydrofuran gelöst. Die Lösung wird bei − 75 °C unter Argon im Verlauf von 10 Minuten tropfenweise mit 11 ml einer 0,95 molaren Lösung von Vinylmagnesiumchlorid in Tetrahydrofuran versetzt. Das kalte Reaktionsgemisch wird in Eis/konz. Ammoniumchlorid eingetragen und mit Aether extrahiert. Der Aetherextrakt wird gewaschen, getrocknet und danach unter vermindertem Druck eingedampft. Das zurückbleibende, zunächst ölige, 5-[4(R)-(−)-Camphanoyloxy-3-oxo-2,6,6-trimethyl-cyclohex-1-en-1-yl]-3-methyl-penta-1,4-dien-3-ol schmilzt nach Reinigung durch Adsorption an Kieselgel und Kristallisation aus Aether/Hexan bei 124-126 °C.

## Beispiel 4

rac. 5-[4-Acetoxy-3-oxo-2,6,6-trimethyl-cyclohex-1-en-1-yl]-3-methyl-penta-1,4-dien-3-ol

Eine aus 0,48 g Magnesium, 2,40 g Aethylbromid und gasförmigem Acetylen hergestellte Lösung von Aethinylmagnesiumbromid in 60 ml Tetrahydrofuran wird bei 0° unter Argon tropfenweise mit einer Lösung von 2,64 g 3-Acetoxy-4-oxo-β-ionon in 10 ml Tetrahydrofuran versetzt. Das Reaktionsgemisch wird nach 25 Minuten in eine eiskalte gesättigte, wässerige Ammoniumchloridlösung eingetragen und mit Aether extrahiert. Der Aetherextrakt wird mit einer gesättigten, wässerigen Natriumchloridlösung neutral gewaschen, über Natriumsulfat getrocknet und unter vermindertem Druck eingedampft. Das zurückbleibende 3-Acetoxy-4-oxo-äthinyl-β-ionol wird durch Adsorption an Kieselgel [Elutionsmittel: Methylenchlorid/Aether 9 : 1] gereinigt und wie folgt weiterverarbeitet:

0,81 g 3-Acetoxy-4-oxo-äthinyl-β-ionol werden in 15 ml Methanol gelöst. Die Lösung wird mit 1,4 ml einer 0,75 %igen Lösung von Dimethyläthanolamin in n-Hexan, sowie mit 1,4 ml einer 0,012 5 %igen Lösung von 1,2-Bis-(2-hydroxyäthylthio)-äthanol in Aether versetzt und nach Zugabe von 25 mg Lindlarkatalysator bei Normaldruck und Raumtemperatur hydriert. Die vom Katalysator abgetrennte Reaktionslösung wird anschliessend unter vermindertem Druck eingedampft. Das zurückbleibende 3-Acetoxy-4-oxo-vinyl-β-ionol [rac. 5-[4-Acetoxy-3-oxo-2,6,6-trimethyl-cyclohex-1-en-1-yl]-3-methyl-penta-1,4-dien-3-ol] ist ein farbloses Oel. $n_D^{24}$ = 1,497 8.

## Beispiel 5

Herstellung von rac. Astaxanthin aus rac. 5-[4-(Phenoxyacetoxy)-3-oxo-2,6,6-trimethyl-cyclohex-1-en-1-yl]-3-methyl-penta-2,4-dien-1-triphenylphosphoniumbromid (IVAB) und 2,7-Dimethyl-octa-2,4,6-trien-1,8-dial (V).

4,4 g rac. 5-[4-(Phenoxyacetoxy)-3-oxo-2,6,6-trimethyl-cyclohex-1-en-1-yl]-3-methyl-penta-2,4-dien-1-triphenylphosphoniumbromid (IVAB) und 0,3 g 2,7-Dimethyl-octa-2,4,6-trien-1,8-dial (V) werden in 70 ml Isopropanol gelöst. Die Lösung wird nach Zusatz von 3,5 ml 2 n Natriummethylat 1 Stunde bei Raumtemperatur gerührt, danach in Wasser eingetragen und mit Methylenchlorid extrahiert. Das nach Eindampfen des Extraktes zurückbleibende rac. Astaxanthin schmilzt nach dem Umkristallisieren aus Chloroform/Methanol oder Pyridin/Wasser bei 216-218 °C.

Die oben eingesetzte Ausgangsverbindung der Formel (IVAB) kann z. B. wie folgt hergestellt werden:

4,5 g rac. 5-[4-(Phenoxyacetoxy)-3-oxo-2,6,6-trimethyl-cyclohex-1-en-1-yl]-3-methyl-penta-1,4-dien-3-ol (IAB) werden in 100 ml Diäthyläther gelöst. Die Lösung wird nach Zugabe von 2 ml Pyridin bei 0° tropfenweise mit 1,2 g Phosphortribromid versetzt. Das Gemisch wird 2 Stunden gerührt, danach in Wasser eingetragen und mit Diäthyläther extrahiert. Der Aetherextrakt wird eingedampft. Das zurückbleibende Bromid wird in Essigsäureäthylester aufgenommen und mit 2,9 g Triphenylphosphin versetzt. Das im Verlauf von 24 Stunden auskristallisierende rac. 2(E), 4(E)-5-[4-Phenoxyacetyl-3-oxo-2,6,6-trimethyl-cyclohex-1-en-1-en-1-yl]-3-methyl-penta-2,4-dien-1-triphenylphosphoniumbromid (IVA) schmilzt bei 158-161 °C.

## Beispiel 6

Herstellung von 3(S), 3'(S)-Astaxanthin aus 5-[4(S)-(Chloracetoxy)-3-oxo-2,6,6-trimethyl-cyclohex-1-en-1-yl]-3-methyl-penta-2,4-dien-1-al (IVA) und 2,7-Dimethyl-octa-2,4,6-trien-1,8-bis-triphenylphosphonium bromid (V):

0,01 Mol Butyllithium in 300 ml Diäthyläther/Hexan [3 : 1] werden bei − 30 °C unter starkem Rühren mit 0,005 Mol 2,7-Dimethyl-octa-2,4,6-trien-1,8-bis-triphenylphosphoniumbromid (V) versetzt. Nach 10 Minuten werden tropfenweise 0,008 Mol 5-[4(S)-(Chloracetoxy)-3-oxo-2,6,6-trimethyl-cyclohex-1-en-1-yl]-3-methyl-penta-2,4-dien-1-al (IVA) in 100 ml Diäthyläther zugegeben. Das Reaktionsgemisch wird 8 Stunden gerührt und anschliessend eingedampft. Das zurückbleibende 3(S), 3'(S)-Astaxanthin-dichloracetat wird in Methanol aufgenommen und unter Rückflussbedingungen zum Sieden erhitzt. Die erhaltene Lösung wird durch Adsorption an Kieselgel gereinigt. Das reine 3(S), 3'(S)-Astaxanthin schmilzt bei 215-217 °C.

Die oben eingesetzten Ausgangsverbindungen der Formel (IVA) kann z. B. wie folgt hergestellt werden:

47,8 g 5-[4(S)-(Chloracetoxy)-3-oxo-2,6,6-trimethyl-cyclohex-1-en-1-yl]-3-methyl-penta-1,4-dien-3-ol (IA) werden in 720 ml Methylenchlorid gelöst. Die Lösung wird nach Zugabe von 200 ml Ameisensäure 45 Minuten bei Raumtemperatur gerührt, danach in Wasser eingetragen und mit Diäthyläther extrahiert. Der Aetherextrakt wird eingedampft. Der Rückstand wird in 500 ml Methanol aufgenommen, mit 100 g Kaliumcarbonat in 250 ml Wasser versetzt, 1 Stunde gerührt, danach in Wasser eingetragen und mit Essigsäureäthylester extrahiert. Das aus dem Extrakt isolierte 5-[4(S)-(Chloracetoxy)-3-oxo-2,6,6-trimethyl-cyclohex-1-en-1-yl]-3-methyl-penta-2,4-dien-1-ol wird wie folgt weiterverarbeitet:

29,7 g 5-[4(S)-(Chloracetoxy)-3-oxo-2,6,6-trimethyl-cyclohex-1-en-1-yl]-3-methyl-penta-2,4-dien-1-ol werden in 3 000 ml Methylenchlorid gelöst. Die Lösung wird nach Zugabe von 1 000 g Mangandioxyd

45 Minuten gerührt und danach filtriert. Nach Abdampfen des Filtrats hinterbleibt 5-[4(S)-(Chloracetoxy)-3-oxo-2,6,6-trimethyl-cyclohex-1-en-1-yl]-3-methyl-penta-2,4-dien-1-al (IVA).

Beispiel 7

Herstellung von 3(R), 3'(R)-Astaxanthin aus 5-[4(R)-(−)-Camphanoyloxy-3-oxo-2,6,6-trimethyl-cyclohex-1-en-1-yl]-3-methyl-penta-2,4-dien-1-triphenylphosphoniumbromid (IVB) und 2,7-Dimethyl-octa-2,4,6-trien-1,8-dial (V).

3,6 g 5-[4(R)-(−)-Camphanoyloxy-3-oxo-2,6,6-trimethyl-cyclohex-1-en-1-yl]-3-methyl-penta-2,4-dien-1-triphenylphosphoniumbromid (IVB) werden unter Argon in 60 ml abs. Isopropanol gelöst. Die Lösung wird nach Zugabe von 300 mg 2,7-Dimethyl-octa-2,4,6-trien-1,8-dial 30 Minuten bei Raumtemperatur gerührt, danach im Verlauf von 10 Minuten tropfenweise mit 2,4 ml einer Lösung von 2 n Natriummethylat in Methanol versetzt. Das Reaktionsgemisch wird zunächst 30 Minuten bei Raumtemperatur, danach 1 Stunde bei 0° gerührt und anschliessend unter Inertgas filtriert. Das auf dem Filter zurückbleibende 3(R), 3'(R)-Astaxanthin schmilzt nach Reinigung durch Adsorption an Kieselgel [Elutionsmittel : Methylenchlorid/Aether/Methanol 89 : 10 : 1] und Kristallisation aus Methylenchlorid/Methanol bei 216-219 °C.

Die oben eingesetzte Ausgangsverbindung der Formel IVB kann z. B. wie folgt hergestellt werden :

2,8 g 5-[4(R)-(−)-Camphanoyloxy-3-oxo-2,6,6-trimethyl-cyclohex-1-en-1-yl]-3-methyl-penta-1,4-dien-3-ol (IC) werden in 30 ml Methylenchlorid gelöst. Die Lösung wird nach Zugabe von 2,28 g Triphenylphosphinhydrobromid 3 Stunden unter Argon bei Raumtemperatur gerührt. Die Reaktionslösung wird eingeengt und mit Essigsäureäthylester versetzt. Das ausfallende rohe 5-[4(R)-(−)-Camphanoyloxy-3-oxo-2,6,6-trimethyl-cyclohex-1-en-1-yl]-3-methyl-penta-2,4-dien-1-triphenylphosphoniumbromid wird ohne weitere Reinigung wie oben beschrieben weiterverarbeitet.

Beispiel 8

22,2 g rac. 3-Hydroxy-4-oxo-β-ionon werden in 210 ml Isopropenylmethyläther gelöst und auf 0 °C gekühlt. Man gibt unter Rühren 1 mg p-Toluolsulfonsäure zu, entfernt das Eisbad und rührt 30 Minuten weiter unter gleichzeitiger Aufwärmung auf Raumtemperatur. Nun werden 5 Tropfen Triäthylamin sowie 50 ml Methylenchlorid zugegeben und unter vermindertem Druck eingedampft. Kristallisation des Rückstandes aus wenig Methylenchlorid/Hexan liefert 26,9 g farbloses rac.-6-(1-Methoxy-1-methyläthoxy)-2,4,4-trimethyl-3-(3-oxo-1-butenyl)-2-cyclohexen-1-on, Smp. 107-109 °C.

14,72 g rac.-6-(1-Methoxy-1-methyläthoxy)-2,4,4-trimethyl-3-(3-oxo-1-butenyl)-2-cyclohexen-1-on werden in 200 ml absolutem Tetrahydrofuran gelöst, und auf etwa − 70 °C gekühlt, wobei ein Teil des Eduktes wieder ausfällt. Nun werden unter Rühren tropfenweise während 45 Minuten 35 ml einer 16,9 %igen Vinylmagnesiumchloridlösung in Tetrahydrofuran zugegeben. Man lässt während etwa 30 Minuten auf − 45 °C erwärmen, wobei das Reaktionsgemisch homogen wird. Dann wird mit Aether extrahiert und der Eindampfrückstand in Hexan aufgenommen. Kristallisation bei etwa − 15 °C ergibt 3-(3-Hydroxy-3-methyl-1,4-pentadienyl)-6-(1-methoxy-1-methyläthoxy)-2,4,4-trimethyl-2-cyclohexen-1-on ; Smp. 64-67 °C.

Das bei der obigen Umsetzung erhaltene Reaktionsgemisch wird in ein Gemisch von 150 ml 1-n wässriger Salzsäure und 300 ml halbkonzentrierter Kochsalzlösung gegossen, dann wird 30 Minuten gerührt und mit Aether extrahiert. Das nach Entfernung des Lösungsmittels zurückbleibende zunächst ölige 6-Hydroxy-3-(3-hydroxy-3-methyl-1,4-pentadienyl)-2,4,4-trimethyl-2-cyclohexen-1-on wird durch Adsorption an Kieselgel (Elutionsmittel : Aether-Hexan) gereinigt. Aus Aether-Hexan-Gemischen lässt es sich bei 0 °C sehr langsam zu farblosen Kristallen vom Smp. 47,5-49 °C kristallisieren.

Zu einer im Eisbad gekühlten Lösung von 5,9 g öligem 6-Hydroxy-3-(3-hydroxy-3-methyl-1,4-pentadienyl)-2,4,4-trimethyl-2-cyclohexen-1-on in 40 ml Methylenchlorid werden 7,14 ml 63 %ige Bromwasserstoffsäure so zugesetzt, dass die Temperatur unter 5 °C bleibt. Man setzt 200 ml Aethylacetat zu und wäscht die organische Phase einmal mit 100 ml halbkonzentrierter Kochsalzlösung, zweimal mit je 100 ml halbkonzentrierter Natriumbicarbonatlösung, trocknet über Natriumsulfat, setzt 0,2 ml Butylenoxid zu und engt die Bromidlösung im Wasserstrahlvakuum bei 30 °C Badtemperatur auf 120 ml ein. Man tropft unter Rühren zu einer Lösung von 6,18 g Triphenylphosphin in 40 ml Aethylacetat und 0,2 ml Butylenoxid, rührt 15 Stunden bei Raumtemperatur und filtriert. Man erhält 10,2 g rac. 5-[(4-Hydroxy)-3-oxo-2,6,6-trimethyl-cyclohex-1-en-1-yl]-3-methylpenta-2,4-dien-1-triphenylphosphoniumbromid (Smp. 172-175 °C), (nach Umkristallisation aus einem Gemisch von 50 ml Methylenchlorid und 100 ml Aethylacetat bei 0 °C 8,8 g vom Smp. 178-180 °C), das durch Umsetzung mit 2,7-Dimethyl-octa-2,4,6-trien-1,8-dial in rac. Astaxanthin übergeführt wird.

**Ansprüche** (für die Vertragstaaten : BE, CH, DE, FR, GB, IT, NL)

1. Verbindungen der Formel

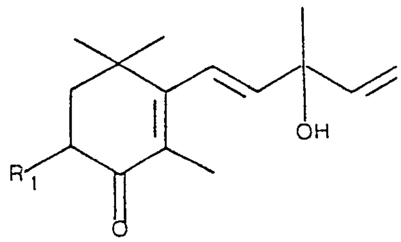

(I)

in der $R_1$ eine in die Hydroxygruppe überführbare Acyloxygruppe oder Aethergruppe darstellt.

2. Verfahren zur Herstellung der Verbindungen der Formel I in Anspruch 1, dadurch gekennzeichnet, dass man eine rac. oder optisch aktive Verbindung der allgemeinen Formel

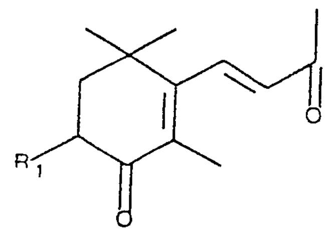

(II).

in der $R^1$ obige Bedeutung hat, vinyliert, oder äthinyliert und partiell hydriert.

3. Verfahren nach Anspruch 2, dadurch gekennzeichnet, dass man als Ausgangsmaterial eine Verbindung der Formel II verwendet, worin $R_1$ eine in die Hydroxygruppe überführbare Acyloxygruppe bedeutet.

4. Verfahren nach Anspruch 2 oder 3, dadurch gekennzeichnet, dass man die in einem aprotischen Lösungsmittel gelöste Ausgangsverbindung der Formel II in einem unter 0° liegenden Temperaturbereich mit einem Vinylmagnesiumhalogenid umsetzt.

5. Verfahren nach Anspruch 2 oder 3, dadurch gekennzeichnet, dass man die in einem aprotischen Lösungsmittel gelöste Ausgangsverbindung der Formel II in einem unter oder über 0° liegenden Temperaturbereich mit einem Aethinylmagnesiumhalogenid umsetzt.

6. Verfahren nach Anspruch 4, dadurch gekennzeichnet, dass man die Ausgangsverbindung der Formel II in Tetrahydrofuran löst und in einem zwischen etwa −30 und etwa −100 °C liegenden Temperaturbereich, vorzugsweise bei etwa −80 °C, mit Vinylmagnesiumchlorid umsetzt.

7. Verfahren nach Anspruch 5, dadurch gekennzeichnet, dass man die Ausgangsverbindung der Formel II in Tetrahydrofuran löst und in einem zwischen etwa −30 bis etwa +30 °C liegenden Temperaturbereich, vorzugsweise bei etwa −0°, mit Aethinylmagnesiumbromid umsetzt.

8. Verfahren nach einem der Ansprüche 2, 5 oder 7, dadurch gekennzeichnet, dass man die Aethinylgruppe des intermediär erhaltenen Reaktionsprodukts zur Vinylgruppe hydriert.

9. Verfahren nach Anspruch 8, dadurch gekennzeichnet, dass man das intermediär erhaltene Reaktionsprodukt in Aethanol löst und mit Hilfe eines partiell vergifteten Palladiumkatalysators bei Normaldruck und Raumtemperatur hydriert.

10. Verwendung einer Verbindung der Formel I in Anspruch 1 in einem Verfahren zur Herstellung von Astaxanthin, wobei man die Verbindung der Formel I in einer für die Kondensation mit einem entsprechenden $C_{10}$-Baustein geeigneten $C_{15}$-Baustein überführt und letzteren in bekannter Weise mit dem $C_{10}$-Baustein zu Astaxanthin kondensiert.

**Ansprüche** (für den Vertragsstaat AT)

1. Verfahren zur Herstellung von Verbindungen der Formel

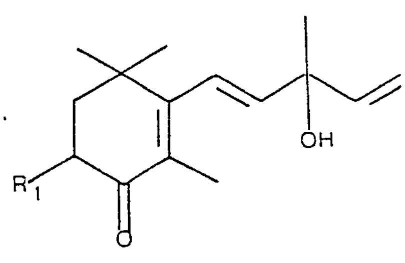

(I)

in der $R_1$ eine in die Hydroxygruppe überführbare Acyloxygruppe oder Aethergruppe darstellt, dadurch gekennzeichnet, dass man eine rac. oder optisch aktive Verbindung der allgemeinen Formel

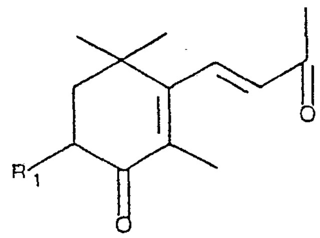

(II)

in der $R_1$ obige Bedeutung hat, vinyliert, oder äthinyliert und partiell hydriert.

2. Verfahren nach Anspruch 1, dadurch gekennzeichnet, dass man als Ausgangsmaterial eine Verbindung der Formel II verwendet, worin $R_1$ eine in die Hydroxygruppe überführbare Acyloxygruppe bedeutet.

3. Verfahren nach Anspruch 1 oder 2, dadurch gekennzeichnet, dass man die in einem aprotischen Lösungsmittel gelöste Ausgangsverbindung der Formel II in einem unter 0° liegenden Temperaturbereich mit einem Vinylmagnesiumhalogenid umsetzt.

4. Verfahren nach Anspruch 1 oder 2, dadurch gekennzeichnet, dass man die in einem aprotischen Lösungsmittel gelöste Ausgangsverbindung der Formel II in einem unter oder über 0° liegenden Temperaturbereich mit einem Aethinyl-magnesiumhalogenid umsetzt.

5. Verfahren nach Anspruch 3, dadurch gekennzeichnet, dass man die Ausgangsverbindung der Formel II in Tetrahydrofuran löst und in einem zwischen etwa −30 und etwa −100 °C liegenden Temperaturbereich, vorzugsweise bei etwa −80 °C, mit Vinylmagnesiumchlorid umsetzt.

6. Verfahren nach Anspruch 4, dadurch gekennzeichnet, dass man die Ausgangsverbindung der Formel II in Tetrahydrofuran löst und in einem zwischen etwa −30 bis etwa +30 °C liegenden Temperaturbereich, vorzugsweise bei etwa −0°, mit Aethinylmagnesiumbromid umsetzt.

7. Verfahren nach einem der Ansprüche 1, 4 oder 6, dadurch gekennzeichnet, dass man die Aethinylgruppe des intermediär erhaltenen Reaktionsprodukts zur Vinylgruppe hydriert.

8. Verfahren nach Anspruch 7, dadurch gekennzeichnet, dass man das intermediär erhaltene Reaktionsprodukt in Aethanol löst und mit Hilfe eines partiell vergifteten Palladiumkatalysators bei Normaldruck und Raumtemperatur hydriert.

9. Verwendung einer Verbindung der Formel I in Anspruch 1 in einem Verfahren zur Herstellung von Astaxanthin, wobei man die Verbindung der Formel I in einer für die Kondensation mit einem entsprechenden $C_{10}$-Baustein geeigneten $C_{15}$-Baustein überführt und letzteren in bekannter Weise mit dem $C_{10}$-Baustein zu Astaxanthin kondensiert.

**Claims** (for the Contracting States : BE, CH, DE, FR, GB, IT, NL)

1. Compounds of the formula

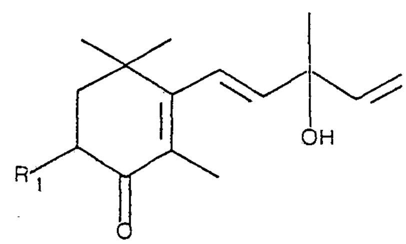

(I)

in which $R_1$ represents an acyloxy group or ether group convertible into the hydroxy group.

2. Process for the manufacture of the compounds of formula I in claim 1, characterized by vinylating, or ethynylating and partially hydrogenating, a rac. or optically active compound of the general formula

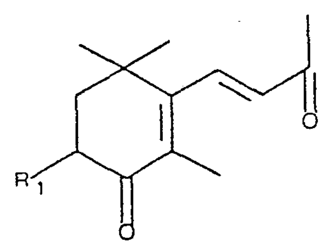

(II)

in which $R^1$ has the above significance.

3. Process according to claim 2, characterized in that as the starting material there is used a compound of formula II wherein $R_1$ signifies an acyloxy group convertible into the hydroxy group.

4. Process according to claim 2 or 3, characterized in that the starting compound of formula II dissolved in an aprotic solvent is reacted with a vinylmagnesium halide in a temperature range lying below 0°.

5. Process according to claim 2 or 3, characterized in that the starting compound of formula II dissolved in an aprotic solvent is reacted with an ethynylmagnesium halide in a temperature range lying below or above 0°.

6. Process according to claim 4, characterized in that the starting compound of formula II is dissolved in tetrahydrofuran and reacted with vinylmagnesium chloride in a temperature range lying between about − 30 and about − 100 °C, preferably at about − 80 °C.

7. Process according to claim 5, characterized in that the starting compound of formula II is dissolved in tetrahydrofuran and reacted with ethynylmagnesium bromide in a temperature range lying between about − 30 to about + 30 °C, preferably at about − 0°.

8. Process according to any one of claims 2, 5 or 7, characterized in that the ethynyl group of the intermediately obtained reaction product is hydrogenated to the vinyl group.

9. Process according to claim 8, characterized in that the intermediately obtained reaction product is dissolved in ethanol and hydrogenated with the aid of a partially poisoned palladium catalyst at normal pressure and room temperature.

10. Use of a compound of formula I in claim 1 in a process for the manufacture of astaxanthin, whereby the compound of formula I is converted into a $C_{15}$-building brick suitable for the condensation with a corresponding $C_{10}$-building brick and the $C_{15}$-building brick is condensed in a known manner with the $C_{10}$-building brick to astaxanthin.

**Claims** (for the Contracting State AT)

1. Process for the manufacture of compounds of the formula

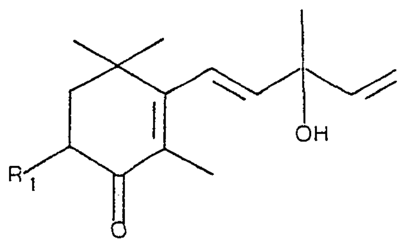

(I)

in which $R_1$ represents an acyloxy group or ether group convertible into the hydroxy group, characterized by vinylating, or ethynylating and partially hydrogenating, a rac. or optically active compound of the general formula

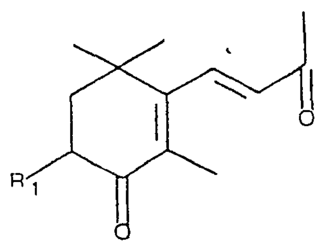

(II)

in which $R^1$ has the above significance.

2. Process according to claim 1, characterized in that as the starting material there is used a compound of formula II wherein $R_1$ signifies an acyloxy group convertible into the hydroxy group.

3. Process according to claim 1 or 2, characterized in that the starting compound of formula II dissolved in an aprotic solvent is reacted with a vinylmagnesium halide in a temperature range lying below 0°.

4. Process according to claim 1 or 2, characterized in that the starting compound of formula II dissolved in an aprotic solvent is reacted with an ethynylmagnesium halide in a temperature range lying below or above 0°.

5. Process according to claim 3, characterized in that the starting compound of formula II is dissolved in tetrahydrofuran and reacted with vinylmagnesium chloride in a temperature range lying between about − 30 and about − 100 °C, preferably at about − 80 °C.

6. Process according to claim 4, characterized in that the starting compound of formula II is dissolved in tetrahydrofuran and reacted with ethynylmagnesium bromide in a temperature range lying between about − 30 to about + 30 °C, preferably at about 0°.

7. Process according to any one of claims 1, 4 or 6, characterized in that the ethynyl group of the intermediately obtained reaction product is hydrogenated to the vinyl group.

8. Process according to claim 7, characterized in that the intermediately obtained reaction product is dissolved in ethanol and hydrogenated with the aid of a partially poisoned palladium catalyst at normal pressure and room temperature.

9. Use of a compound of formula I in claim 1 in a process for the manufacture of astaxanthin, whereby the compound of formula I is converted into a $C_{15}$-building brick suitable for the condensation with a corresponding $C_{10}$-building brick and the $C_{15}$-building brick is condensed in a known manner with the $C_{10}$-building brick to astaxanthin.

**Revendications** (pour les Etats Contractants : BE, CH, DE, FR, GB, IT, NL)

1. Composés de formule

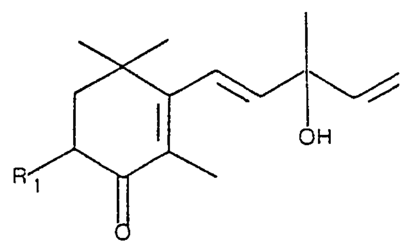

(I)

dans laquelle $R_1$ représente un groupe acyloxy ou un groupe éther convertible en le groupe hydroxy.

2. Procédé de préparation des composés de formule I de la revendication 1, caractérisé en ce que l'on soumet un composé racémique ou énantiomère de formule générale

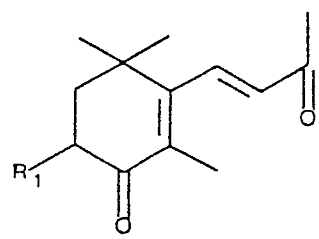

(II)

dans laquelle $R^1$ a la signification indiquée ci-dessus, à vinylation ou éthynylation et hydrogénation partielle.

3. Procédé selon la revendication 2, caractérisé en ce que l'on utilise en tant que produit de départ un composé de formule II dans laquelle $R_1$ représente un groupe acyloxy convertible en le groupe hydroxy.

4. Procédé selon la revendication 2 ou 3, caractérisé en ce que l'on fait réagir le composé de départ de formule II en solution dans un solvant aprotonique, dans un intervalle de température inférieur à 0°, avec un halogénure de vinyl-magnésium.

5. Procédé selon la revendication 2 ou 3, caractérisé en ce que l'on fait réagir le composé de départ de formule II en solution dans un solvant aprotonique, dans un intervalle de température inférieur ou supérieur à 0°, avec un halogénure d'éthynyl-magnésium.

6. Procédé selon la revendication 4, caractérisé en ce que l'on dissout le composé de départ de formule II dans le tétrahydrofuranne et on le fait réagir dans un intervalle de température situé entre − 30 et − 100 °C environ, de préférence au voisinage de − 80 °C, avec du chlorure de vinyl-magnésium.

7. Procédé selon la revendication 5, caractérisé en ce que l'on dissout le composé de départ de formule II dans le tétrahydrofuranne et on le fait réagir dans un intervalle de température situé entre − 30 et + 30 °C environ, de préférence au voisinage de 0°, avec le bromure d'éthynyl-magnésium.

8. Procédé selon l'une des revendications 2, 5 ou 7, caractérisé en ce que l'on hydrogène en groupe vinyle le groupe éthynyle du produit de réaction obtenu en intermédiaire.

9. Procédé selon la revendication 8, caractérisé en ce que l'on dissout le produit de réaction obtenu en intermédiaire dans l'éthanol et on l'hydrogène à pression normale et température ambiante à l'aide d'un catalyseur au palladium partiellement empoisonné.

10. Utilisation d'un composé de formule I de la revendication 1 dans un procédé de préparation de l'astaxanthine dans lequel on convertit le composé de formule I en un élément de structure en $C_{15}$ approprié à la condensation avec un élément de structure en $C_{15}$ approprié à la condensation avec un élément de structure correspondant en $C_{10}$ et on forme l'astaxanthine par condensation de cet élément, de manière connue en soi, avec l'élément de structure en $C_{10}$.

**Revendications** (pour l'Etat Contractant AT)

1. Procédé de préparation des composés de formule

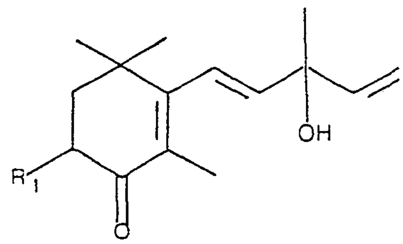

(I)

dans laquelle $R_1$ représente un groupe acyloxy ou un groupe éther convertible en le groupe hydroxy, caractérisé en ce que l'on soumet un composé racématique ou énantiomère de formule générale

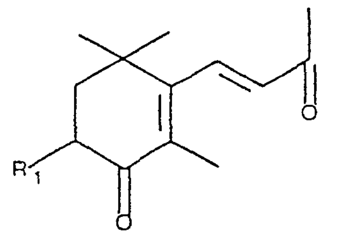

(II)

dans laquelle $R^1$ a la signification indiquée ci-dessus, à vinylation ou éthynylation et hydrogénation partielle.

2. Procédé selon la revendication 1, caractérisé en ce que l'on utilise en tant que produit de départ un composé de formule II dans laquelle $R_1$ représente un groupe acyloxy convertible en le groupe hydroxy.

3. Procédé selon la revendication 1 ou 2, caractérisé en ce que l'on fait réagir le composé de départ de formule II en solution dans un solvant aprotonique, dans un intervalle de température inférieur à 0°, avec un halogénure de vinyl-magnésium.

4. Procédé selon la revendication 1 ou 2, caractérisé en ce que l'on fait réagir le composé de départ de formule II en solution dans un solvant aprotonique, dans un intervalle de température inférieur ou supérieur à 0°, avec un halogénure d'éthynyl-magnésium.

5. Procédé selon la revendication 3, caractérisé en ce que l'on dissout le composé de départ de formule II dans le tétrahydrofuranne et on le fait réagir dans un intervalle de température situé entre − 30 et − 100 °C environ, de préférence au voisinage de − 80 °C, avec du chlorure de vinyl-magnésium.

6. Procédé selon la revendication 4, caractérisé en ce que l'on dissout le composé de départ de formule II dans le tétrahydrofuranne et on le fait réagir dans un intervalle de température situé entre − 30 et + 30 °C environ, de préférence au voisinage de 0°, avec le bromure d'éthynyl-magnésium.

7. Procédé selon l'une des revendications 1, 4 ou 6, caractérisé en ce que l'on hydrogène en groupe vinyle le groupe éthynyle du produit de réaction obtenu en intermédiaire.

8. Procédé selon la revendication 7, caractérisé en ce que l'on dissout le produit de réaction obtenu en intermédiaire dans l'éthanol et on l'hydrogène à pression normale et température ambiante à l'aide d'un catalyseur au palladium partiellement empoisonné.

9. Utilisation d'un composé de formule I de la revendication 1 dans un procédé de préparation de l'astaxanthine dans lequel on convertit le composé de formule I en un élément de structure en $C_{15}$ approprié à la condensation avec un élément de structure correspondant en $C_{10}$ et on forme l'astaxanthine par condensation de cet élément, de manière connue en soi, avec l'élément de structure en $C_{10}$.